# EUROPEAN PATENT SPECIFICATION

(11) **EP 2 210 091 B1**
(45) Date of publication and mention of the grant of the patent: **07.12.2011**
(21) Application number: 08805131.3
(22) Date of filing: 08.10.2008
(51) Int. Cl.: G01N 33/36, G01N 21/29, D06H 3/02

(54) **FABRIC COLOUR GUIDE**
TEXTILFARBEN-LEITFADEN
NUANCIER DE TISSU

(30) Priority: 23.11.2007 EP 07121379
(43) Date of publication of application: 28.07.2010
(73) Proprietor: Unilever PLC, London EC4Y 0DY (GB); Unilever N.V., 3013 AL Rotterdam (NL)
(72) Inventor: MORLEY, Nicola-Jane, Wirral Merseyside CH63 3JW (GB); SINGLETON, Stephen, John, Wirral Merseyside CH63 3JW (GB)
(74) Representative: Hardy, Susan Margaret
(86) International application number: PCT/EP2008/063439
(87) International publication number: WO 2009/065663

(56) References cited:
- EP-A- 0 927 787
- WO-A1-2008/064977
- CH-A5- 679 338
- GB-A- 2 358 404
- US-A- 2 785 958
- US-B1- 6 416 612

## Description

The present invention concerns a fabric colour guide for monitoring colour of a fabric.

In many circumstances, fabric colour is extremely valuable to consumers. Indeed, for some garments (e.g. expensive coloured suit shirts, uniforms) precise colour is necessary so that dulling of the colour is perceived as unacceptable. In these cases, reduced colour may even result in the garment being discarded by the consumer. Repeated wash-wear cycles can reduce the colour of a garment.

The ability of washing powders to maintain and even rejuvenate colour may be increased by the use of shading dyes.

An objective is to provide a device and method for in-home consumer monitoring of the changes in colour of fabric and evaluation of the colour benefits of a laundry composition.

The present invention provides a package containing a laundry composition comprising an agent for improving, rejuvenating or maintaining colour in combination with a fabric colour guide comprising at least one colour or range of colours.

The fabric colour guide allows in-home consumer monitoring of the change in colour of a fabric by comparing the shade of the fabric with the colour displayed visually on the guide.

The guide may comprise a colour scale which displays a colour/range of colours varying progressively from low colour at one end of the scale to high colour at the other end of the scale.

Alternatively or additionally the guide may comprise a colour palette of different colours. The colours may be grouped according to hue, lightness, position in the spectrum or they may be in random order.

The or each colour may be defined in terms of L*a*b* values (the CIE LAB colour space, (CIE 1976 L* a* b*), where L* is the lightness value of the colour (L*=O yields black and L*=100 yields white), a* is the red-green value (negative values indicate green while positive values indicate red) and b* is the yellow-blue value (negative values indicate blue and positive values indicate yellow.

The variation in colour is preferably due to a change in colouration (a* and/or b*) and/or change in luminance/lightness (L*).

The highest colour may be defined as L*ₘₐₓ, a*ₘₐₓ, b*ₘₐₓ (UV-included) such that:
L*ₘₐₓ is in the range **1-70**, preferably 5-50
a*ₘₐₓ is in the range -40 to +40, preferably -20.0 to + 20.0
b*ₘₐₓ is in the range +40 to -40, preferably +20.0 to -20.0. and the minimum colour values as L*ₘᵢₙ, a*ₘᵢₙ, b*ₘᵢₙ such that $ΔL * = L ⁢ {*}_{min} - L ⁢ {*}_{max} ,$
where ΔL* is 3 to 30 units preferably 5 to 10 units, reflecting the luminance of the coloured cloth $Δa * = a ⁢ {*}_{min} - a ⁢ {*}_{max} ,$
where Δa* is +/- (0 to 30) units, preferably +/- (0 to 10) units reflecting red/green value of the colour of the cloth $Δb * = b ⁢ {*}_{min} - b ⁢ {*}_{max} ,$
where Δb* is +/- (0 to 30) units, preferably +/- (0 to 10) units reflecting the blue/yellow value of the colour of the cloth.

The a* values may be constant or at least within +/- 1 unit, to restrict the guide to focus on blue-yellow colouration rather than red-green.

The b* values may be constant or at least within +/- 1 unit, to restrict the guide to focus on red-green colouration rather than blue-yellow.

The L* valves may be constant or at least within +/-1 unit to restrict the guide to focus on red-green and/or blue-yellow i.e. colour proper rather than on lightness or luminance.

For dark colours a preferable range of L*ₘₐₓ, a*ₘₐₓ, b*ₘₐₓ (UV-included) are:
L*ₘₐₓ is in the range 1-30, preferably 5-15
a*ₘₐₓ is in the range -8 to +8, preferably -4.0 to + 4.0
b*ₘₐₓ is in the range +10 to -15, preferably 0 to -10.

ΔL* is preferably 3 to 30 units preferably 5 to 10 units.

Δa* is preferably +/- (0 to 10) units, preferably
+/- (0 to 5) units.

Δb* is preferably +/-(0 to 30) units preferably
+/- (0 to 10).

Intervening colour values in any scale/palette or in any part of a scale/palette may be between the above extremes. The intervening colour values may be equally distributed. Alternatively, the L* and b* and a* values may be lowered in alternate steps.

The guide may be configured for monitoring changes in colour due to an increase or decrease in one particular colour (e.g. blue). Accordingly, the b* and/or a* values may vary in either the positive or negative with the L* values (and either a* or b* values corresponding with any colours not being measured) maintained constant or constant at least within +/- 1 unit.

The guide may be configured for monitoring changes in colour due only to the change in luminance or lightness of a fabric. Accordingly, the L* values may be varied and the a* values and b * values maintained constant to within +/- 1 unit. The a* may be maintained at zero or at least close to zero (within +/- 1.0 and preferably within +/- 0.3 of zero). The b* values may be maintained constant at a value (to within +/- 1 unit) which is less than 2 and preferably less than +/-3 units.
There may be multiple guides each showing different changes in one or more in different colours and lightness, so the consumer can select the appropriate guide according to the a particular colouration/whiteness of the coloured fabric to undergo the measuring. In this way the consumer can identify and appreciate the cause of a change in colour and/or lightness, by matching the fabric with the correct scale.

The colour palette or scale may be continuous (where colour changes gradually) across the guide or scale it may be stepped. In the case of a stepped scale, this will comprise a plurality of discrete portions, wherein the progression from one portion of the scale to another portion, involves a step-change in colour and/or lightness.

In the case of a stepped palette or scale the total colour difference between adjacent shades is preferably such that the shade of one portion of the scale is easily and quickly distinguished by the naked eye from the shade of an adjacent portion. This means that in-home testing can be done by the busy consumer, without the need for laboratory conditions or equipment. However, at the same time it is preferred that the scale is focussed on subtle changes in colour within a restricted range of high colour values.

Accordingly, the difference in colour between adjacent portions may be defined such that the colour of each portion has Lab values: L*ₙ, a*ₙ, b*ₙ, defined by : ${L}_{n}^{*} = {L}_{max}^{*} + \frac{\left(n-1\right)}{\left(N-1\right)} ⁢ Δ ⁢ L *$ ${a}_{n}^{*} = {a}_{max}^{*} + \frac{\left(n-1\right)}{\left(N-1\right)} ⁢ Δ ⁢ a *$ ${b}_{n}^{*} = {b}_{max}^{*} + \frac{\left(n-1\right)}{\left(N-1\right)} ⁢ Δ ⁢ b *$
where the highest colour has n=1, there are N discrete shades of whites in the scale and the shades decrease in colour in equal steps (+/- 0.3 units) along the 3 axes.

Preferably, the total colour difference represented as the ΔE value (corresponding to the positive square root of [(L*ₙ-L*ₙ₋₁)²+(a*ₙ- a*ₙ₋₁)²+(b*ₙ- b*ₙ₋₁)²] is preferably greater than or equal to 0.5, and more preferably 0.5 to 2.0, (UV-included).

The values of L* and b* may vary alternately on the scale, such L* only changes when n is an even number and b* when n is an odd number.

The values of L* and b* may vary alternately on the scale, such b* only changes when n is an even number and L* when n is an odd number.

The L* value may decrease linearly whereas the b* values decrease for even values of n.

The b* value may decrease linearly with each value of n, whereas the L* values are only decreased for even values of n.

The values of L* and a* may vary alternately on the scale, such L* only changes when n is an even number and a* when n is an odd number.

The values of L* and a* may vary alternately on the scale, such a* only changes when n is an even number and L* when n is an odd number.

The L* value may decrease linearly whereas the b* values decrease for even values of n.

The a* value may decrease linearly with each value of n, whereas the L* values are only decreased for even values of n.

The portions may have a uniform shade of colour. A uniformly shaded portion is easier for the consumer to match with the fabric.

By "continuous", it is meant that the change in colour appears (to the naked eye) continuous along the scale or scales. A continuous scale(s) may however, include demarcation by visual indicia e.g. lines, so as to divide up the scale into multiple portions. This makes it easier for the consumer to remember, without recording, roughly where the colour of the fabric falls on the scale.

The guide may be labelled with directional visual indicia to direct the user to hold the product in a predetermined orientation. The higher colour values may be on one side i.e., the right or left of the user (when facing the product).

The portions of the scale or palette may be labelled with numbers, names, logos etc. applied by e.g. printing to ease monitoring.

The portions of the scale or palette may be arranged in a single, straight row. Alternatively, the portions may be arranged in multiple rows. The row or rows may be straight or curved.

Preferably the portions are of equal size and shape. This is advantageous in that no portion/s is/are given undue preference due to size. This affords more accurate comparison between the colour of the portions and the fabric based on colour.

The portions may be 0.5 to 4 cm, and may be greater than 2 cm in length / diameter. By length/diameter it is meant the greatest length/diameter of a shape e.g. this will be the diameter of a circle, the greater diameter of an ellipse, the length of a side of a square, and the length of the longer side of a rectangle.

The colour scale or palette is preferably a rectangle and is preferably 1-4cm, and may be greater than 2 cm by 10-12 cm.

The guide may comprise apertures corresponding with the scale or palette, whereby the fabric can be viewed through the apertures to identify the level of colour according to the scale or palette.

If the guide includes apertures, it is preferred that the length or diameter of the portions are at least 1.3 times, more preferably two times the length or diameter of the apertures. Preferably the portions are at least 1 cm in diameter and more preferably at least 3cm. This allows a large area of the guide to surround the aperture for matching with the fabric to be tested.

The apertures may correspond with the portions, and there may be one aperture per portion. Alternatively there may be more than one aperture per portion so that the colour of the fabric is viewed through multiple apertures, which may be in a pattern or grid. The aperture or apertures may be entirely enclosed within the perimeter of the or each portion.

Alternatively or additionally, one or more of the apertures may be in the form of a shape which is cut into the perimeter of the portion i.e. so that it is not enclosed by the perimeter, but itself forms part of the perimeter.

The aperture may have a curved perimeter. It may be circular or elliptical. A curved perimeter is advantageous as it can make judging the shade easier for the human eye.

The aperture may have an undulating perimeter, and the undulations may be such that the shape has multiple (2-dimensional) protrusions.

Alternatively the shape may be angular, such as star shaped.

Visually interesting shapes such as undulating and star shapes provide visual stimulae for the consumer, to attract the consumer to do the testing and also providing visual stimulae during testing.

The colour guide may have 3-20 portions, preferably 8-14 portions. A more complicated guide offers greater accuracy however excessively complicated guides can be off-putting for some consumers. Surprisingly a very simple guide increases the frequency of use of the device for first time users, and monitoring becomes more habitual. There may be a choice of scales, a first scale having 3-20 portions and at least one other scale having a lower number, such as half, than the first scale.

The guide may have less than 10 portions. This is advantageous for colour guides focussed on a restricted range of colour, as described above. This is the optimum range which provides portions which are easily visually distinguished by the consumer.

The guide may be a planar member. By planar, it is meant less that 2mm thick, preferably less than 1 mm. This has the advantage that the surface carrying the scale is not distanced (by thickness) from the fabric substrate during measuring, which allows for more accurate comparisons.

Preferably the guide is sufficiently flexible so it can flex to lie against a flexible substrate such as fabric. This has the advantage that the guide can be easily flexed to conform to the shape of a garment to carry out the colour comparison.

The colour guide may be provided with the packaging, unattached and loose inside. This has the advantage that the guide is quickly obtained on opening the package. The colour guide may be wrapped in packaging to protect it from the washing composition, so that when it is initially retrieved from the pack and used, it is not contaminated with the washing composition which could then transfer on to the fabric during colour measuring.

Alternatively the guide may be integral with the packaging, e.g. printed on the side of the pack. This reduces packaging material, prevents the guide being accidentally lost and also the consumer is reminded to use the guide each time they dose from the pack.

The step of comparing may take place after washing with a laundry composition. Alternatively or additionally, it may also take place before washing. This allows the consumer to evaluate the efficacy of the washing process to improve colour. The step of comparing may be repeated e.g. before and/or after multiple e.g. consecutive washing operations, for evaluation of the progressive improvement in colour of a particular washing composition. The guide can confirm the maintenance of colour due to a particular washing composition.

The invention also allows the consumer to compare the different washing compositions or brands of compositions or other conditions such as temperature of the wash. The step of comparing may take place after other events which affect washing, for instance drying of washed fabrics outside, in sunlight etc.

Accordingly the invention provides a package containing a laundry composition in combination with a fabric colour guide of the first aspect, including any optional advantageous features as described above, and preferably together with instructions for use of said fabric colour guide to measure the colour of a fabric according to the method of the second aspect including any optional, advantageous features as described above.

The provision of a colour guide together with the washing powder enables the consumer to effectively monitor the effectiveness of the powder and appreciate the benefit of colour agents added to the washing powder. Thus the consumer is given more control over the evaluation of different products and different washing and also drying conditions.

The guide may be perfumed, and the perfume of the guide may be the same as or correspond with the perfume of the laundry composition.

The detergent powder may contain an agent for improving rejuvenating or maintaining colour. The agent may comprise one or more dyes for increasing perceived colour. Preferably the colour/s of the guide correspond with the colour of the/or each dye whereby increase in the perceived colour can be measured/monitored using said guide using the human eye.

Preferably the dye, or dyes together, has/have a peak absorption wavelength on the substrate fabric of 540 nm to 650 nm, and further preferably from 570 nm to 630 nm.

Dyes that are substantive to fabrics may be used.
The dyes may be a direct dye so as to be substantive to cotton or they may be disperse and solvent dyes which are substantive to synthetic fibres e.g., polyester and nylon. The composition may contain a mixture of dye so as to be substantive to both fibres.

The laundry composition may contain predominately anionic surfactants. In this case dyes containing acid groups are preferred. For use in products which contain predominantly cationic surfactants, dyes containing basic groups are preferred. This is to prevent precipitation between the dye and surfactant.

Suitable dyes for use in products containing predominately anionic surfactants include those listed in the Colour Index as Direct Violet Dyes Direct Blue dyes, Acid Blue and Acid Violet dyes.

Dyes which may be metabolised to carcinogenic amines should not be used. For example dyes which when reduced release benzidene, 3,3'-dimethoxybenzidene, 3,3'-dimethybenzidene or 3,3'-dichlorobenzidene should not be used.

The laundry composition may contain predominately cationic surfactants. Suitable dyes here include those listed in the Colour Index as Basic Blue and Basic Violet Dyes.

The dyes may comprise one or more hydrophobic dyes selected from benzodifuranes, methine, triphenylmethanes, napthalimides, pyrazole, napthoquinone, anthraquinone and mono-azo or di-azo dyes. Hydrophobic dyes are dyes which do not contain any charged water solubilising group. Hydrophobic dyes may be selected from the groups of disperse and solvent dyes. Blue and violet anthraquinone and mono-azo dye are preferred.

Preferred dyes include solvent violet 13, disperse violet 27 disperse violet 26, disperse violet 28, disperse violet 63 and disperse violet 77

The shading dye(s) may be a direct violet dye. These are particularly useful for for cotton containing fabrics Preferred are dyes are selected from the group comprising bis-azo direct violet dyes of the formula: 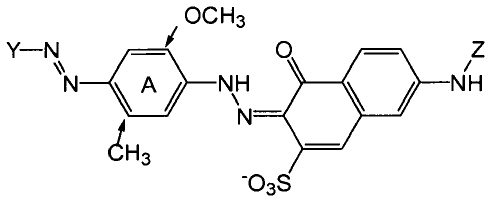 where Z is H or phenyl, the A ring is preferably substituted by a methyl and methoxy group at the positions indicated by arrows, the A ring may also be a naphthyl ring, the Y group is a phenyl or naphthyl ring, which may be substituted by sulphonate groups and may be mono or disubstituted by methyl groups.

The shading dye(s) may comprise the dyes direct violet 7, direct violet 9, direct violet 11, direct violet 26, direct violet 31, direct violet 35, direct violet 40, direct violet 41, direct violet 51, and direct violet 99.

Cu containing direct dyes such as direct violet 66 may also be used.

The shading dye(s) may comprise acid dyes for shading cotton and may be selected from acid blue 98, acid violet 17, acid violet 50, acid black 1, acid red 51, acid red 17 acid blue 29. One preferred acid shading dye is acid blue 98.

The shading dye may comprise a photobleach covalently linked to another blue or violet chromophore.
The shading dye may comprise a reactive dye covalently linked to a polymer or a solid particle.

Pigments may be included to shade clothes Dyes and pigments are listed in the Color Index International published by Society of Dyers and Colourists and the American Association of Textile Chemists and Colorists.

The shading dye may be a pigment.

Preferred pigments are pigment blue 1, 1:2, 1:3, 2, 2:1, 2:2, 3, 4, 5, 7, 9, 10, 10:1, 11, 12, 13, 14, 15, 15:1, 15:2, 15:3, 15:4, 15:6, 16, 18, 19, 20, 21, 22, 23, 25, 26, 27, 28, 29, 30, 31, 32, 34, 35, 36, 56, 57, 58, 59, 60, 61, 61:1, 62, 63, 64, 65, 66, 67, 69, 71, 72, 73, 74, 75, 79, 80, 83 and pigment violet 1, 1:1, 1:2, 2, 3, 3:1, 3:3, 3:4, 5, 5:1, 7:1, 8, 9, 11, 12, 13, 14, 15, 16, 18, 19, 23, 25, 27, 28, 29, 31, 32, 35, 37, 39, 41, 42, 43, 44, 45, 47, 48, 50, 54, 55 and 56

More Preferred organic pigments are pigment violet 1, 1:1, 1:2, 2, 3, 5:1, 13, 23, 25, 27, 31, 37, 39, 42, 44, 50 and Pigment blue 1, 2, 9, 10, 14, 18, 19, 24:1, 25, 56, 60, 61, 62, 66, 75, 79 and 80.

More preferred pigments are pigment violet 3, 13, 23, 27, 37, 39, pigment blue 14, 25, 66 and 75.

The most preferred is pigment violet 23.

The shading dye may comprise a dye adsorbed onto a solid particle, such as a clay.

For ease of incorporation into laundry products it is preferred if the shading dye is supplied in a liquid form. The level of dye in the laundry composition may be in the range from 0.000001 wt % to 0.01 wt % preferably in the range from 0.0001 to 0.01 wt%.

The composition may comprise a fluorescent agent (optical brightener). Fluorescent agents are available commercially. The fluorescent agent may be supplied and used in the form of their alkali metal salts, for example, the sodium salts. The total amount of the fluorescent agent or agents used in the composition may be generally from 0.005 to 2 wt %, preferably 0.01 to 0.1 wt %.

Preferred classes of fluorescer are: Di-styryl biphenyl compounds, e.g. Tinopal (Trade Mark) CBS-X, Di-amine stilbene di-sulphonic acid compounds, e.g. Tinopal DMS pure Xtra and Blankophor (Trade Mark) HRH, and Pyrazoline compounds, e.g. Blankophor SN. Preferred fluorescers are: sodium 2 (4-styryl-3-sulfophenyl)-2H-napthol[1,2-d]trazole, disodium 4,4'-bis{[(4-anilino-6-(N methyl-N-2 hydroxyethyl) amino 1,3,5-triazin-2-yl)]amino}stilbene-2-2' disulfonate, disodium 4,4'-bis{[(4-anilino-6-morpholino-1,3,5-triazin-2-yl)]amino} stilbene-2-2' disulfonate, and disodium 4,4'-bis(2-sulfoslyryl)biphenyl.

The laundry composition and colour guide may include a common shading dye or fluorescer, as described above.

Such compositions are preferably laundry detergent compositions used for washing (especially particulate detergents, liquid detergents, laundry bars, pastes, gels or tablets), laundry fabric conditioners used for softening fabrics, pretreatment products, post-treatment products, tumble dryer products, ironing products etc. Preferably they are laundry treatment products which are applied in an aqueous environment.

Various non-limiting embodiments of the invention will now be more particularly described with reference to the following figures in which:
Figure 1 shows a packaged laundry composition according to the invention where the package may be a flexible bag; and
flexible bag; and
Figure 2 shows a colour guide with apertures including a loose or removable colour guide.

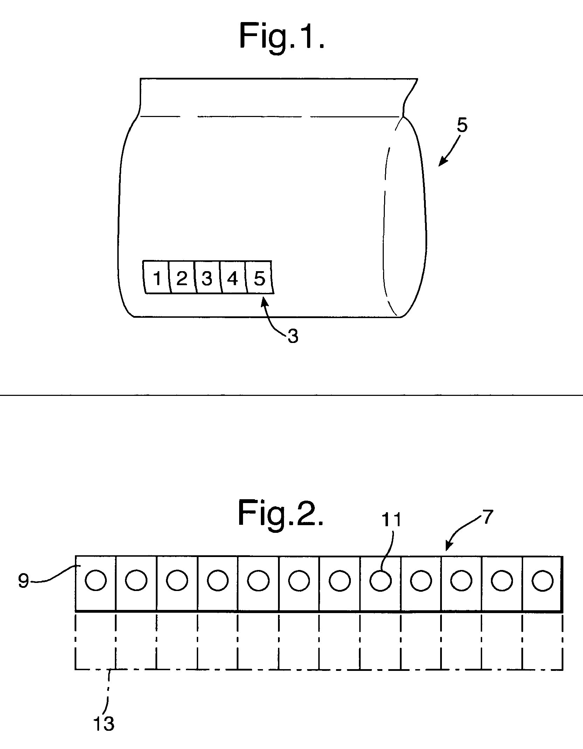

Referring to figure 1, a flexible plastic bag 5 is shown. The bag 5 has a colour scale printed onto the outside. The scale consists of five 2 x 2cm² square portions, numbered 1,2,3,4,5 from left to right. The portions have the following L*,a*,b* values measured using a reflectometer with UV included:

| | **L*** | **a*** | **b*** |
|---|---|---|---|
| **1** | 10.0 | 0.5 | -4 |
| **2** | 12.0 | 0.5 | -2 |
| **3** | 14.0 | 0.5 | 0 |
| **4** | 16.0 | 0.5 | +2 |
| **5** | 18.0 | 0.5 | +4 |

The pack contains 1kg of laundry composition A or B or C or D.

A plastic pack as described above in reference to the figure except that the portions have the following LAB values measured using a reflectometer with UV included:

| | L* | a* | b* |
|---|---|---|---|
| 1 | 10.0 | 0.5 | -4 |
| 2 | 12.0 | 0.5 | -4 |
| 3 | 12.0 | 0.5 | -2 |
| 4 | 16.0 | 0.5 | -2 |
| 5 | 16.0 | 0.5 | 0 |

The pack contains a laundry composition A or B or C or D.

The above packages are provided with colour scales printed onto a cardboard strip. The strip is provided loose within or removable from the package 5 so that it can be retrieved on first opening by the consumer. The portions may be 3 x 3 cm and may be as shown in figure 2.

The above cardboard strip 7 is as shown in figure 2 and is 20 cm long and 6 cm wide. At the top of the card 7 in a space 2 cm long and the width of the card, a tradename is printed (not shown). The rest of the card is equally divided into boxes of 3 x 3 cm, so that there are 12 boxes in total. The colour of the boxes are as follows:

| | L* | a* | b* |
|---|---|---|---|
| 1 | 8.5 | 1 | -4 |
| 2 | 9 | 0.7 | -4 |
| 3 | 9.5 | 0.4 | -4 |
| 4 | 10 | 0.1 | -4 |
| 5 | 10.5 | -0.2 | -4 |
| 6 | 11 | -0.5 | -4 |
| 7 | 11.5 | -0.8 | -4 |
| 8 | 12.0 | -1.1 | -4 |
| 9 | 12.5 | -1.4 | -4 |
| 10 | 13.0 | -1.7 | -4 |
| 11 | 13.5 | -2.0 | -4 |
| 12 | 14.0 | -2.3 | -4 |

A circular hole 11 of 1cm radius is cut into the center of each portion.

The circular hole 11 allows the consumer to view the fabric within an window surrounded by an area of the colour shade of the scale. The size of the hole 11 relative to the size of the portion is selected so that there is sufficient area of the colour shade to enable quick, accurate matching of the fabric with the colour shade.

The portions may be arranged in a single row. Alternatively the portions are in two rows (shown at dotted line B).

| **Formulation** | **A** | **B** | **C** | **D** |
|---|---|---|---|---|
| NaLAS | 14 | 10 | 15 | 21 |
| NI (7EO) | 10 | 5 | 21 | 15 |
| SLES (3EO) | 7 | 10 | 7 | - |
| Soap | 2 | 4 | 1 | 0 |
| Citric acid | 1 | 1 | - | 1 |
| glycerol | 0 | 1 | 5 | 0 |
| Propylene glycol | 5 | 3 | 0 | 4 |
| Sodium chloride | 1 | - | - | - |
| Amine ethoxylated polymers | 0.5 | 1 | - | - |
| Triethanol amine | 0 | 0.5 | 3 | 1 |
| perfume | 0.2 | 0.1 | 0.3 | 0.4 |
| fluorescer | 0.05 | 0.1 | 0.15 | 0.2 |
| Protease | 0.005 | 0.01 | - | 0.005 |
| Amylase | 0.001 | 0.003 | - | |
| lipase | - | 0.003 | - | - |
| Fluorescer | 0.1 | 0.15 | 0.05 | 0.3 |
| Direct Violet 9 | 0.0006 | 0.0008 | | 0.0004 |
| Direct Violet 99 | - | - | 0.0002 | - |
| Solvent Violet 13 | - | 0.02 | 0 | 0.01 |
| Water/impurities/mi nors | remainder | remainder | remainder | remainder |

Enzyme levels are given as percent pure enzyme. Levels of direct violet 9, direct violet 99, solvent violet 13 and Sulfonated Zn Pthalocyanine photobleach are given as pure dye. NI (7EO) refers to R-(OCH₂CH₂)ₙOH, where R is an alkyl chain of C12 to C15, and n is 7.

The formulations are prepared by adding direct violet 9, direct violet 99 and the Sulfonated pigment violet 23 into the slurry which is then spray dried. Alternatively, the dyes may be added via post-dosed MgSO₄ granules.

The solvent violet 13 was dissolved in non-ionic surfactant (7E0) and granulated onto zeolite, to give a granule containing 0.2wt% dye. This was post-dosed to the formulation.

## Claims

1. A package containing a laundry composition comprises an agent for improving, rejuvenating or maintaining colour in combination with a fabric colour guide comprising at least one colour or at least range of colours together with instructions for use of said fabric colour guide to measure the colour of a fabric according to a method of consumer-measurement of the colour of fabric, including the step of comparing the fabric with the colour guide.

2. A package according to claim 1 wherein the colour/range of colours are defined in terms of L* a* b* values and the range of L*ₘₐₓ, a*ₘₐₓ, b*ₘₐₓ (UV-included) are:
L*ₘₐₓ is in the range 1-30, preferably 5-15
a*ₘₐₓ is in the range -8 to +8, preferably -4.0 to + 4.0
b*ₘₐₓ is in the range +10 to -15, preferably 0 to -10.

3. A package according to any preceding claim wherein the guide is configured for measurement of one particular colour whereby a* and/or b* values vary and L* are maintained constant.

4. A package according to any preceding claim wherein the method includes the step of comparing is repeated before and/or after multiple washing operations.

5. A package according to any preceding claim wherein the laundry composition comprises one or more dyes for increasing the perceived colour.

6. A package according to any preceding claim claim wherein said dye/dyes has/have a peak absorption wavelength on the substrate fabric of 540nm to 650 nm.

7. A package according to any preceding claim claim wherein said dye/dyes has/have a peak absorption wavelength on the substrate fabric of 570nm to 630 nm.

8. A package according to any preceding claim wherein the colour/s of the guide correspond with the colour/s of the dye/s.

## Patentansprüche

1. Packung, die ein Waschmittel enthält, umfassend ein Mittel zur Verbesserung, Auffrischung oder Aufrechterhaltung von Farbe in Kombination mit einem Gewebe-Farbleitfaden, umfassend wenigstens eine Farbe oder wenigstens einen Bereich von Farben zusammen mit Instruktionen zur Verwendung des Gewebe-Farbleitfadens, um die Farbe eines Gewebes nach einem Verfahren der Konsumentenmessung der Farbe von Gewebe zu messen, das den Schritt des Vergleichens des Gewebes mit dem Farbleitfaden umfasst.

2. Packung gemäß Anspruch 1, wobei die Farbe/die Farbbereiche als L*-, a*-, b*-Werte definiert sind und die Bereiche für L*ₘₐₓ, a*ₘₐₓ, b*ₘₐₓ (UV eingeschlossen) sind:
L*ₘₐₓ ist im Bereich 1-30, vorzugsweise 5-15,
a*ₘₐₓ ist im Bereich -8 bis +8, vorzugsweise -4,0 bis +4,0,
b*ₘₐₓ ist im Bereich +10 bis -15, vorzugsweise 0 bis -10.

3. Packung gemäß einem vorangehenden Anspruch, wobei der Leitfaden zur Messung einer bestimmten Farbe konfiguriert ist, wobei a*- und/oder b*-Wert(e) variiert/ variieren und L* konstant gehalten wird.

4. Packung gemäß einem vorangehenden Anspruch, wobei das Verfahren den Schritt des Vergleichens umfasst, der vor und/oder nach mehreren Waschprozessen wiederholt wird.

5. Packung gemäß einem vorangehenden Anspruch, wobei das Waschmittel einen Farbstoff oder mehrere Farbstoffe zur Verstärkung der wahrgenommenen Farbe umfasst.

6. Packung gemäß einem vorangehenden Anspruch, wobei der Farbstoff/die Farbstoffe eine Spitzen-Absorptionswellenlänge auf dem Substratgewebe von 540 nm bis 650 nm hat/haben.

7. Packung gemäß einem vorangehenden Anspruch, wobei der Farbstoff/die Farbstoffe eine Spitzen-Absorptionswellenlänge auf dem Substratgewebe von 570 nm bis 630 nm hat/haben.

8. Packung gemäß einem vorangehenden Anspruch, wobei die Farbe(n) des Leitfadens der Farbe/den Farben des Farbstoffe/der Farbstoffe entspricht.

## Revendications

1. Conditionnement contenant une composition de lessive comprenant un agent pour améliorer, rajeunir ou maintenir la couleur en combinaison avec un guide de couleur de tissu comprenant au moins une couleur ou au moins une gamme de couleurs avec des instructions d'utilisation dudit guide de couleur de tissu pour mesurer la couleur d'un tissu selon un procédé de mesure par le client de la couleur d'un tissu.

2. Conditionnement selon la revendication 1, dans lequel la couleur/gamme de couleurs sont définies en termes de valeurs L*, a*, b* et les gammes de L*ₘₐₓ, a*ₘₐₓ, b*ₘₐₓ (ultraviolets inclus) sont :
L*ₘₐₓ est dans la plage de 1 à 30, de préférence de 5 à 15
a*ₘₐₓ est dans la plage de -8 à +8, de préférence de -4,0 à +4,0
b*ₘₐₓ est dans la plage de +10 à -15, de préférence de 0 à -10.

3. Conditionnement selon l'une quelconque des revendications précédentes, dans lequel le guide est configuré pour mesurer une couleur particulière, moyennant quoi les valeurs a* et/ou b* varient et les valeurs L* sont maintenues constantes.

4. Conditionnement selon l'une quelconque des revendications précédentes, dans lequel le procédé consiste en ce que l'étape de comparaison est répétée avant et/ou après de multiples opérations de lavage.

5. Conditionnement selon l'une quelconque des revendications précédentes, dans lequel la composition de lessive comprend un ou plusieurs colorants pour augmenter la couleur perçue.

6. Conditionnement selon l'une quelconque des revendications précédentes, dans lequel ledit ou lesdits colorants ont une longueur d'onde d'absorption crête sur le tissu de substrat de 540 nm à 650 nm.

7. Conditionnement selon l'une quelconque des revendications précédentes, dans lequel ledit ou lesdits colorants a/ont une longueur d'onde d'absorption crête sur le tissu de substrat de 570 nm à 630 nm.

8. Conditionnement selon l'une quelconque des revendications précédentes, dans lequel les couleurs du guide correspondent aux couleurs des colorants.
